# EUROPEAN PATENT APPLICATION

(11) **EP 4 130 256 A1**
(43) Date of publication of application: **08.02.2023**
(21) Application number: 21832203.0
(22) Date of filing: 03.03.2021
(51) Int. Cl.: C12N 9/16, C12N 9/12, C40B 50/06

(54) **ENZYME REACTION SOLUTION FOR CONSTRUCTING SEQUENCING LIBRARY AND USE THEREOF**

(30) Priority: 28.06.2020 CN 202010599034
(71) Applicant: Jiangsu Conquers Medical Technology Co., Ltd., Taizhou, Jiangsu 225309 (CN); Nanjing Vazyme Biotech Co., Ltd., Nanjing, Jiangsu 210046 (CN)
(72) Inventor: NIE, Junwei, Nanjing, Jiangsu 210046 (CN); QU, Zhipeng, Nanjing, Jiangsu 210046 (CN); ZHANG, Lijun, Nanjing, Jiangsu 210046 (CN); HAN, Jinxiong, Nanjing, Jiangsu 210046 (CN); JIANG, Mingyang, Nanjing, Jiangsu 210046 (CN); QIU, Cui, Nanjing, Jiangsu 210046 (CN)
(74) Representative: Pfenning, Meinig & Partner mbB
(86) International application number: PCT/CN2021/078865
(87) International publication number: WO 2022/001149

(57) **Abstract**

Provided in the present invention is an enzyme reaction solution for constructing a sequencing library and the use thereof. The enzyme reaction solution comprises an enzyme composition and a reaction buffer, wherein the enzyme composition comprises a nucleic acid endonuclease, a DNA polymerase, and a polynucleotide kinase; and the reaction buffer comprises a metal salt, a substrate, and a buffer medium aqueous solution. The present application aims to optimize the formulation of an enzyme reaction solution. The cleavage, terminal repair and addition of A to the terminal of a nucleic acid sample is achieved by a one-step reaction. In a suitable buffer system, the enzyme digestion reaction rate and the terminal repair reaction rate reach a balance. In the case where the initial amount of the sample is 100 pg to 1 µg and the processing time is the same, a sequencing library with a consistent length distribution is obtained.

## Description

### TECHNICAL FIELD

The present application belongs to the field of biotechnology, and relates to an enzyme reaction solution for constructing a sequencing library and application thereof.

### BACKGROUND

The high-throughput sequencing market is growing rapidly as the cost of high-throughput sequencing has plummeted. DNA sequencing accounts for about half of the entire high-throughput sequencing market, and thus there is an increasingly urgent need for a simple, fast, and high-throughput DNA library construction technology. The first and most critical step in the process of DNA library construction is to fragment large-fragment DNA molecules into small DNA fragments of a specific length without bias. At present, the mainstream DNA fragmentation method is ultrasonic mechanical fragmentation. However, the ultrasonic mechanical fragmentation instrument is often expensive, and many industrial, scientific research, and medical laboratories can not be equipped with this instrument; the operation of ultrasonic mechanical DNA fragmentation is complex and time-consuming, and requires real-time sampling to detect the fragmentation effect of samples; in addition, the number of ultrasonic mechanical fragmented samples is small, and the input of single sample treatment is small, which can not meet the demand for high-throughput sample treatment in the industrial and medical fields. Fragmentase is capable of solving the above problems well. The enzymatic method-based DNA sample treatment method is capable of being completed in a thermal cycler, and the operation process is simple and fast; fragmentase is capable of performing non-specific random cleavage of DNA molecules, and thus it is expected to replace the traditional ultrasonic mechanical method to fragment large-fragment DNA molecules into small-fragment DNA molecules of a specific length without bias, so as to be applied to DNA library construction in high-throughput sequencing.

Currently available enzymatic method-based DNA fragmentation technologies comprise: 1) randomly generating a nick on a double-stranded DNA molecule using Vvn, and simultaneously cutting another DNA molecule at the nick site using T7 endonuclease; 2) breaking the double-stranded DNA in the presence of Mn²⁺ or Mg²⁺ using DNaseI; and 3) mixing a variety of restriction endonucleases (e.g., MspI, AluI, CviQI, MseI, MlucI, HaeIII, and the like) that recognize specific base sequences to perform cleavage of the DNA molecule.

At present, the initial input of samples for DNA library construction varies from 100 pg to 1 µg, with a difference of 5 orders of magnitude. However, the commercial kits on the market are not enough to meet the market demand in terms of initial amount compatibility. On the one hand, the lower compatibility limit of commercial kits is not less than 100 pg, and on the other hand, different initial amounts require different reaction time. In an actual experimental process, the amounts of nucleic acids obtained from different samples are different, and it is inevitable to use samples with different initial amounts for library construction. Therefore, the reaction time needs to be regulated for different initial amounts of samples, which not only increases the complexity of the experiment and the demand for instruments, but also limits the application of the library construction method in large-throughput samples.

CN108998434A discloses a fragmentase fragmentation buffer and a method for improving the fragmentase fragmentation efficiency. The fragmentase fragmentation buffer comprises a buffer component suitable for the fragmentase to function, PEG8000 at a final concentration of not more than 10%, and Mg²⁺ at a final concentration of 10 to 25 mM. By adding different proportions of PEG and Mg²⁺ to the reaction buffer system to regulate the efficiency and resultant fragment size of DNA fragmentation, a fragmentation buffer that can meet the requirements for the PE100 and PE150 paired-end sequencing libraries on the BGISEQ-500 platform is obtained, but it is still not compatible with samples with an initial amount of 100 pg to 1 µg.

Therefore, it is necessary to improve the existing library construction kits.

### SUMMARY

The present application provides an enzyme reaction solution for constructing a sequencing library and application thereof. By optimizing the type and dosage of the enzyme composition, and cooperating with the metal ions and the buffer medium in the reaction buffer, the enzyme reaction solution balances the enzyme digestion reaction rate and the end repair reaction rate in the library construction process, and achieves the technical effect of obtaining a sequencing library of a consistent length under the conditions of different initial amounts of samples and the same treatment time, which has wide applicability and convenient operability.

In a first aspect, the present application provides an enzyme reaction solution for constructing a sequencing library, comprising an enzyme composition and a reaction buffer; wherein
the enzyme composition comprises an endonuclease, a DNA polymerase, and a polynucleotide kinase; and
the reaction buffer comprises a metal salt, a substrate, and an aqueous buffer medium solution.

The enzyme composition of the present application comprises an endonuclease for cleavage, a DNA polymerase for end repair and 3' A-tailing, and a polynucleotide kinase for 5' phosphorylation. The enzyme composition is in one reaction system, and the cleavage, end repair and A-tailing of nucleic acid samples are realized through one-step reaction. The reaction buffer comprises not only an ionic buffer required for the cleavage reaction, but also an ionic buffer required for end repair, as well as substrate molecules dNTPs, dATP and ATP required for various reactions. Various components cooperate with each other within a certain concentration range to balance the enzyme digestion reaction rate and the end repair reaction rate, thereby achieving the technical effect of obtaining a sequencing library of a consistent length with the initial amount of samples of 100 pg to 1 µg and the same treatment time.

The present application uses an endonuclease to perform cleavage on a nucleic acid sample, and the endonuclease may be, for example, any one or a combination of at least two of endonuclease dsDNase, T7 endonuclease, salt-active endonuclease SAN, endonuclease Vvn or endonuclease DNaseI.

In some embodiments, the endonuclease may be, for example, any one or a combination of at least two of shrimp-derived endonuclease dsDNase, T7 phage-derived T7 endonuclease, salt-active endonuclease SAN, *Vibrio vulnificus*-derived endonuclease Vvn or bovine pancreas-derived endonuclease DNaseI.

In some embodiments, the final concentration of the endonuclease in the enzyme reaction solution is 0.003 to 0.05 U/µL, and may be, for example, 0.003 U/µL, 0.004 U/µL, 0.005 U/µL, 0.006 U/µL, 0.007 U/µL, 0.008 U/µL, 0.009 U/µL, 0.01 U/µL, 0.02 U/µL, 0.03 U/µL, 0.04 U/µL or 0.05 U/µL, preferably 0.004 to 0.03 U/µL, and further preferably 0.005 to 0.01 U/µL.

In some embodiments, when the endonuclease is Vvn, the final concentration of the endonuclease Vvn in the enzyme reaction solution is 0.1 to 0.5 ng/µL, and may be, for example, 0.1 ng/µL, 0.2 ng/µL, 0.3 ng/µL, 0.4 ng/µL or 0.5 ng/µL.

The present application regulates the cleavage rate of a nucleic acid by an endonuclease by optimizing the concentration of the endonuclease in the reaction system and controlling the dosage of the endonuclease for different types of endonucleases.

In the present application, a DNA polymerase is used to perform end repair on the cleaved nucleic acid or to perform an A-tailing reaction at the 3' terminal of the cleaved nucleic acid. The DNA polymerase comprises a low-temperature DNA polymerase and/or a thermostable DNA polymerase, wherein the low-temperature DNA polymerase may be, for example, any one or a combination of at least two of T4 phage-derived DNA polymerase, T7 phage-derived DNA polymerase, *E*. *coli*-derived DNA polymerase I or the large fragment Klenow of *E*. *coli*-derived DNA polymerase I, which performs end repair on the cleaved nucleic acid; and the thermostable DNA polymerase may be, for example, Taq DNA polymerase, which performs an A-tailing reaction at the 3' terminal of the cleaved nucleic acid, so that the subsequent adaptor ligation reaction is performed based on the TA ligation principle.

In the present application, the term "thermostable DNA polymerase" may be Taq DNA polymerase, the residual activity of which is greater than 90% of the original activity after 2 h of reaction at 70°C, the residual activity of which is 60% of the original activity after 2 h of reaction at 93°C, and the residual activity of which is 40% of the original activity after 2 h of reaction at 95°C; and the term "low-temperature DNA polymerase" refers to a DNA polymerase that does not have thermostable activity relative to the "thermostable DNA polymerase".

In some embodiments, the final concentration of the low-temperature DNA polymerase in the enzyme reaction solution is 0.01 to 0.05 U/µL, and may be, for example, 0.01 U/µL, 0.02 U/µL, 0.03 U/µL, 0.04 U/µL or 0.05 U/µL, and preferably 0.02 to 0.04 U/µL.

In some embodiments, the final concentration of the thermostable DNA polymerase in the enzyme reaction solution is 0.03 to 1.2 U/µL, and may be, for example, 0.03 U/µL, 0.04 U/µL, 0.05 U/µL, 0.06 U/µL, 0.07 U/µL, 0.08 U/µL, 0.09 U/µL, 0.1 U/µL, 0.2 U/µL, 0.3 U/µL, 0.4 U/µL, 0.5 U/µL, 0.6 U/µL, 0.7 U/µL, 0.8 U/µL, 0.9 U/µL, 1.0 U/µL, 1.1 U/µL or 1.2 U/µL, preferably 0.04 to 1.1 U/µL, further preferably 0.05 to 1.0 U/µL, and still preferably 0.06 to 0.9 U/µL.

The present application regulates the end repair rate of a nucleic acid by a DNA polymerase by optimizing the concentration of the DNA polymerase in the reaction system and controlling the dosage of the DNA polymerase for different types of DNA polymerases.

The enzyme reaction solution of the present application further comprises a polynucleotide kinase, which may be, for example, T4 polynucleotide kinase (T4 PNK) for performing 5' phosphorylation on the cleaved nucleic acid. The final concentration of the polynucleotide kinase in the enzyme reaction solution is 0.05 to 0.2 U/µL, and may be, for example, 0.05 U/µL, 0.06 U/µL, 0.07 U/µL, 0.08 U/µL, 0.09 U/µL, 0.1 U/µL or 0.2 U/µL.

In some embodiments, the enzyme composition further comprises an accessory protein.

The present application uses an accessory protein to regulate the cleavage rate of the endonuclease and the end repair rate of the DNA polymerase. The accessory protein comprises a bovine serum albumin (BSA) and/or a single-chain binding protein, and preferably, the single-chain binding protein comprises an *E*. *coli*-derived single-chain binding protein *E. coli* SSB and/or a T4 phage-derived single-chain binding protein T4 GP32.

In some embodiments, the final concentration of the accessory protein in the enzyme reaction solution is 0.05 to 1 µg/µL, and may be, for example, 0.05 µg/µL, 0.06 µg/µL, 0.07 µg/µL, 0.08 µg/µL, 0.09 µg/µL, 0.1 µg/µL, 0.2 µg/µL, 0.3 µg/µL, 0.4 µg/µL, 0.5 µg/µL, 0.6 µg/µL, 0.7 µg/µL, 0.8 µg/µL, 0.9 µg/µL or 1 µg/µL, preferably 0.05 to 0.5 µg/µL, and further preferably 0.1 to 0.4 µg/µL.

The present application regulates the cleavage rate of the endonuclease and the end repair rate of the DNA polymerase by optimizing the concentration of the accessory protein in the reaction system and controlling the dosage of the accessory protein for different types of accessory proteins.

In some embodiments, the metal cation comprises any one or a combination of at least two of Mg²⁺, Mn²⁺, Na⁺ or Ca²⁺, the Mg²⁺-containing salt may be MgCl₂ and/or Mg₂SO₄, the Mn²⁺-containing salt may be MnCl₂, the Na⁺-containing salt may be NaCl, and the Ca²⁺-containing salt may be CaCl₂.

In the present application, by regulating the type and concentration of metal cations in the enzyme reaction solution, the metal cations and the buffer medium jointly regulate the reaction system to be in a suitable acid-base environment, and control the enzymatic activities of the endonuclease and DNA polymerase, so as to balance the enzyme digestion reaction rate and the end repair reaction rate.

In some embodiments, the final concentration of the Mg²⁺ in the enzyme reaction solution is 0 to 20 mM, and may be, for example, 1 mM, 2 mM, 3 mM, 4 mM, 5 mM, 6 mM, 7 mM, 8 mM, 9 mM, 10 mM, 11 mM, 12 mM, 13 mM, 14 mM, 15 mM, 16 mM, 17 mM, 18 mM, 19 mM or 20 mM, preferably 2 to 18 mM, further preferably 3 to 17 mM, and still preferably 4 to 16 mM or 5 to 15 mM.

In some embodiments, the final concentration of the Mn²⁺ in the enzyme reaction solution is 0.05 to 1 mM, and may be, for example, 0.05 mM, 0.06 mM, 0.07 mM, 0.08 mM, 0.09 mM, 0.1 mM, 0.2 mM, 0.3 mM, 0.4 mM, 0.5 mM, 0.6 mM, 0.7 mM, 0.8 mM, 0.9 mM or 1 mM, preferably 0.07 to 0.8 mM, further preferably 0.09 to 0.6 mM, and still preferably 0.1 to 0.5 mM.

In some embodiments, the final concentration of the Na⁺ in the enzyme reaction solution is 0 to 50 mM, and may be, for example, 1 mM, 2 mM, 3 mM, 4 mM, 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 25 mM, 30 mM, 35 mM, 40 mM, 45 mM or 50 mM, preferably 0 to 30 mM, further preferably 5 to 30 mM, and still preferably 5 to 20 mM or 5 to 15 mM.

In some embodiments, the final concentration of the Ca²⁺ in the enzyme reaction solution is 0 to 10 mM, and may be, for example, 1 mM, 2 mM, 3 mM, 4 mM, 5 mM, 6 mM, 7 mM, 8 mM, 9 mM or 10 mM, and preferably 0 to 8 mM, 0 to 5 mM, 0 to 3 mM, 1 to 10 mM, 1 to 5 mM, 1 to 3 mM, 2 to 10 mM or 2 to 5 mM.

In some embodiments, the substrate comprises any one or a combination of at least two of dNTPs, dATP or ATP.

In some embodiments, the final concentration of the dNTPs in the enzyme reaction solution is 0.05 to 0.5 mM, and may be, for example, 0.05 mM, 0.06 mM, 0.07 mM, 0.08 mM, 0.09 mM, 0.1 mM, 0.2 mM, 0.3 mM, 0.4 mM or 0.5 mM, and preferably 0.05 to 0.4 mM, 0.05 to 0.3 mM, 0.05 to 0.2 mM, 0.05 to 0.1 mM, 0.07 to 0.5 mM, 0.07 to 0.4 mM, 0.07 to 0.3 mM, 0.07 to 0.2 mM, 0.07 to 0.1 mM, 0.09 to 0.5 mM, 0.09 to 0.4 mM, 0.09 to 0.3 mM, 0.09 to 0.2 mM or 0.09 to 0.1 mM.

In the present application, the concentration of dNTPs is limited within a certain range, which is beneficial to regulate the end repair rate of the DNA polymerase.

In some embodiments, the final concentration of the dATP in the enzyme reaction solution is 0.1 to 2 mM, and may be, for example, 0.1 mM, 0.2 mM, 0.3 mM, 0.4 mM, 0.5 mM, 0.6 mM, 0.7 mM, 0.8 mM, 0.9 mM, 1 mM, 1.1 mM, 1.2 mM, 1.3 mM, 1.4 mM, 1.5 mM, 1.6 mM, 1.7 mM , 1.8 mM, 1.9 mM or 2 mM, and preferably 0.1 to 1.5 mM, 0.1 to 1 mM, 0.3 to 2 mM, 0.3 to 1.5 mM, 0.3 to 1 mM, 0.5 to 2 mM, 0.5 to 1.5 mM, 0.5 to 1 mM or 0.5 to 0.8 mM.

In some embodiments, the final concentration of the ATP in the enzyme reaction solution is 1 to 10 mM, and may be, for example, 1 mM, 2 mM, 3 mM, 4 mM, 5 mM, 6 mM, 7 mM, 8 mM, 9 mM or 10 mM, and preferably 1 to 9 mM, 1 to 8 mM, 1 to 7 mM, 1 to 6 mM, 1 to 5 mM, 3 to 10 mM, 3 to 9 mM, 3 to 8 mM, 3 to 7 mM or 3 to 6 mM.

In some embodiments, the buffer medium comprises 4-hydroxyethyl piperazine ethanesulfonic acid (HEPES) and/or tris(hydroxymethyl)aminomethane (TRIS).

In some embodiments, the final concentration of the buffer medium in the enzyme reaction solution is 10 to 50 mM, and may be, for example, 10 mM, 15 mM, 20 mM, 25 mM, 30 mM, 35 mM, 40 mM, 45 mM or 50 mM, and preferably 10 to 40 mM, 10 to 30 mM, 15 to 50 mM, 15 to 45 mM, 15 to 40 mM or 15 to 30 mM.

In some embodiments, the pH of the enzyme reaction solution is 7.0 to 8.5, and may be, for example, 7.0, 7.1, 7.2, 7.3, 7.4, 7.5, 7.6, 7.7, 7.8, 7.9, 8.0, 8.1, 8.2, 8.3, 8.4 or 8.5, and preferably 7.5 to 8.1.

In some embodiments, the enzyme composition comprises T4 PNK, BSA, T4 GP32, Vvn, T7 endonuclease, Taq DNA polymerase, and Klenow.

In some embodiments, the enzyme composition comprises T4 PNK, BSA, *E. coli* SSB, dsDNase, Taq DNA polymerase, and Klenow.

In some embodiments, the enzyme composition comprises T4 PNK, BSA, T4 GP32, SAN, Taq DNA polymerase, and *E. coli* DNA polymerase I.

In some embodiments, the enzyme composition comprises T4 PNK, BSA, T4 GP32, DNaseI, Taq DNA polymerase, and T4 DNA polymerase.

In some embodiments, the reaction buffer comprises 4-hydroxyethyl piperazine ethanesulfonic acid (HEPES), MgCl₂, CaCl₂, NaCl, MnCl₂, dNTPs, dATP, and ATP.

In some embodiments, the reaction buffer comprises tris(hydroxymethyl)aminomethane (TRIS), MgCl₂, CaCl₂, NaCl, MnCl₂, dNTPs, dATP, and ATP.

In some embodiments, the enzyme reaction solution further comprises glycerol.

In some embodiments, the enzyme reaction solution comprises tris(hydroxymethyl)aminomethane (TRIS), MgCl₂, CaCl₂, NaCl, MnCl₂, dNTPs, dATP, ATP, T4 PNK, BSA, T4 GP32, DNaseI, Taq DNA polymerase, T4 DNA polymerase, and glycerol.

In some embodiments, the enzyme reaction solution comprises 4-hydroxyethyl piperazine ethanesulfonic acid (HEPES), MgCl₂, CaCl₂, NaCl, MnCl₂, dNTPs, dATP, ATP, T4 PNK, BSA, T4 GP32, DNaseI, Taq DNA polymerase, T4 DNA polymerase, and glycerol.

In some embodiments, the enzyme reaction solution comprises 4-hydroxyethyl piperazine ethanesulfonic acid (HEPES), MgCl₂, CaCl₂, NaCl, MnCl₂, dNTPs, dATP, ATP, T4 PNK, BSA, T4 GP32, DNaseI, Taq DNA polymerase, T4 DNA polymerase, and glycerol.

In some embodiments, the enzyme reaction solution comprises tris(hydroxymethyl)aminomethane (TRIS), MgCl₂, CaCl₂, NaCl, MnCl₂, dNTPs, dATP, ATP, T4 PNK, BSA, T4 GP32, SAN, Taq DNA polymerase, *E. coli* DNA polymerase I, and glycerol.

In some embodiments, the enzyme reaction solution comprises 30 mM tris(hydroxymethyl)aminomethane (TRIS), 15 mM MgCl₂, 5 mM CaCl₂, 10 mM NaCl, 0.1 mM MnCl₂, 0.1 mM dNTPs, 0.6 mM dATP, 1 mM ATP, 0.1 U/µL T4 PNK, 0.3 µg/µL BSA, 0.1 µg/µL T4 GP32, 0.06 U/µL DNaseI, 0.3 U/µL Taq DNA polymerase, 0.9 U/µL T4 DNA polymerase, and glycerol.

In some embodiments, the enzyme reaction solution comprises 20 mM 4-hydroxyethyl piperazine ethanesulfonic acid (HEPES), 15 mM MgCl₂, 5 mM CaCl₂, 10 mM NaCl, 0.5 mM MnCl₂, 0.1 mM dNTPs, 0.6 mM dATP, 1 mM ATP, 0.1 U/µL T4 PNK, 0.3 µg/µL BSA, 0.1 µg/µL T4 GP32, 0.06 U/µL DNaseI, 0.3 U/µL Taq DNA polymerase, 0.9 U/µL T4 DNA polymerase, and glycerol.

In some embodiments, the enzyme reaction solution comprises 20 mM 4-hydroxyethyl piperazine ethanesulfonic acid (HEPES), 5 mM MgCl₂, 5 mM CaCl₂, 10 mM NaCl, 0.1 mM MnCl₂, 0.1 mM dNTPs, 0.6 mM dATP, 1 mM ATP, 0.1 U/µL T4 PNK, 0.3 µg/µL BSA, 0.1 µg/µL T4 GP32, 0.06 U/µL DNaseI, 0.3 U/µL Taq DNA polymerase, 0.9 U/µL T4 DNA polymerase, and glycerol.

In some embodiments, the enzyme reaction solution comprises 30 mM tris(hydroxymethyl)aminomethane (TRIS), 15 mM MgCl₂, 5 mM CaCl₂, 10 mM NaCl, 0.5 mM MnCl₂, 0.1 mM dNTPs, 0.6 mM dATP, 1 mM ATP, 0.1 U/µL T4 PNK, 0.3 µg/µL BSA, 0.1 µg/µL T4 GP32, 0.06 U/µL SAN, 0.3 U/µL Taq DNA polymerase, 0.06 U/µL *E. coli* DNA polymerase I, and glycerol.

In the present application, the type and concentration of metal ions as well as the type and concentration of the buffer medium in the reaction system cooperate with each other within a certain range to maintain the pH value of the enzyme reaction solution in a relatively stable and suitable state, thereby affecting the activities of the endonuclease and DNA polymerase, and ensuring that the enzyme digestion reaction rate and the end repair reaction rate are consistent, so that the length of the constructed sequencing library is suitable for the second-generation sequencing platform.

In a second aspect, the present application provides a method for constructing a sequencing library, comprising:
adding a target nucleic acid into the enzyme reaction solution according to the first aspect, incubating, and performing fragmentation, end repair, 5' phosphorylation and 3' A-tailing of the target nucleic acid;
ligating an incubation product with a sequencing adaptor; and
performing PCR on a ligation product to obtain a sequencing library.

Preferably, the target nucleic acid is added in an amount of 100 pg to 1 µg, and may be, for example, 100 pg, 1 ng, 10 ng, 100 ng or 1 µg.

Preferably, the incubation conditions involve keeping at 35 to 40°C for 10 to 20 min, and at 60 to 70°C for 20 to 40 min, and preferably keeping at 37°C for 15 min, and at 65°C for 30 min.

In a third aspect, the present application provides a kit for constructing a sequencing library, comprising the enzyme reaction solution according to the first aspect.

Preferably, the kit further comprises any one or a combination of at least two of a sequencing adaptor, a ligation reaction reagent or a PCR reagent.

It can be understood by those skilled in the art that the reaction buffer and the enzyme composition in the enzyme reaction solution of the present application may be separately packaged, and the final concentration of the reaction buffer and the enzyme composition after mixing is consistent with or substantially consistent with that described in the present application, and this situation is also within the protection scope of the present application.

In a fourth aspect, the present application provides a sequencing library, which is constructed using the method according to the second aspect or the kit according to the third aspect; and the length of the sequencing library is 300 to 500 bp.

Compared with the prior art, the present application has the following beneficial effects:
(1) the present application integrates fragmentation, end repair, 3' dA-tailing, and 5' phosphorylation modification of nucleic acid samples into a one-step reaction; the enzyme composition comprises an endonuclease for cleavage, a DNA polymerase for end repair and 3' A-tailing, and a polynucleotide kinase for 5' phosphorylation; these components and the reaction buffer cooperate with each other within a certain concentration range to control the enzyme digestion reaction rate and the end repair reaction rate, thereby achieving the technical effect of obtaining a sequencing library of a consistent length with the initial amount of samples of 100 pg to 1 µg and the same treatment time, and improving the success rate of library construction; and
(2) the enzyme reaction solution of the present application further simplifies the enzymatic method-based DNA fragmentation and library construction methods, and has wider applicability and more convenient operability.

### BRIEF DESCRIPTION OF DRAWINGS

Fig. 1 shows the length distribution of the sequencing library constructed using the enzyme reaction solution of combination 1;
Fig. 2 shows the length distribution of the sequencing library constructed using the enzyme reaction solution of combination 2;
Fig. 3 shows the length distribution of the sequencing library constructed using the enzyme reaction solution of combination 3;
Fig. 4 shows the length distribution of the sequencing library constructed using the enzyme reaction solution of combination 4;
Fig. 5 shows the length distribution of the sequencing library constructed using the enzyme reaction solution of combination 5;
Fig. 6 shows the length distribution of the sequencing library constructed using the enzyme reaction solution of combination 6;
Fig. 7 shows the length distribution of the sequencing library constructed using the enzyme reaction solution of combination 7;
Fig. 8 shows the length distribution of the sequencing library constructed using the enzyme reaction solution of combination 8;
Fig. 9 shows the length distribution of the sequencing library constructed using the enzyme reaction solution of combination 9;
Fig. 10 shows the length distribution of the sequencing library constructed using the enzyme reaction solution of combination 10;
Fig. 11 shows the length distribution of the sequencing library constructed using the enzyme reaction solution of combination 11;
Fig. 12 shows the length distribution of the sequencing library constructed using the enzyme reaction solution of combination 12;
Fig. 13 shows the length distribution of the sequencing library constructed using the enzyme reaction solution of combination 13;
Fig. 14 shows the length distribution of the sequencing library constructed using the enzyme reaction solution of combination 14;
Fig. 15 shows the length distribution of the sequencing library constructed using the enzyme reaction solution of combination 15;
Fig. 16 shows the length distribution of the sequencing library constructed using the enzyme reaction solution of combination 16.

### DETAILED DESCRIPTION

In order to further illustrate the technical means adopted in the present application and its effects, the present application will be further described below with reference to the examples and the drawings. It should be understood that the specific implementations described herein are only used to explain the present application, but not to limit the present application.

Where no specific technique or condition is indicated in the examples, the procedures should be carried out in accordance with the techniques or conditions described in the literature in the art, or in accordance with the product manuals. The used reagents or instruments without indication of the manufacturers are all conventional products that can be commercially purchased through regular channels.

### Example 1. Preparation of enzyme reaction solution

In this example, an enzyme reaction solution was first prepared, including two portions: the reaction buffer as shown in Table 1 and the enzyme composition as shown in Table 2. The reaction buffer in Table 1 and the enzyme composition in Table 2 were cross-combined to obtain enzyme reaction solutions with different formulations as shown in Table 3. The manufacturer and Cat# of protease are shown in Table 4, and the amino acid sequence of *Vibrio vulnificus* nuclease Vvn is shown in SEQ ID NO: 1.

**Table 1. Reaction buffer**

| A | | B | | C | | D | |
|---|---|---|---|---|---|---|---|
| Compo nent | Concentr ation | Compo nent | Concentr ation | Compo nent | Concentr ation | Compo nent | Concentr ation |
| HEPES | 200 mM | HEPES | 200 mM | Tris | 300 mM | Tris | 300 mM |
| MgCl₂ | 150 mM | MgCl₂ | 50 mM | MgCl₂ | 150 mM | MgCl₂ | 150 mM |
| CaCl₂ | 30 mM | CaCl₂ | 30 mM | CaCl₂ | 30 mM | CaCl₂ | 30 mM |
| NaCl | 100 mM | NaCl | 100 mM | NaCl | 100 mM | NaCl | 100 mM |
| MnCl₂ | 5 mM | MnCl₂ | 1 mM | MnCl₂ | 5 mM | MnCl₂ | 1 mM |
| dNTPs | 1 mM | dNTPs | 1 mM | dNTPs | 1 mM | dNTPs | 1 mM |
| dATP | 6 mM | dATP | 6 mM | dATP | 6 mM | dATP | 6 mM |
| ATP | 50 mM | ATP | 50 mM | ATP | 50 mM | ATP | 50 mM |

**Table 2. Enzyme composition**

| Enzyme composition 1 | | Enzyme composition 2 | | Enzyme composition 3 | | Enzyme composition 4 | |
|---|---|---|---|---|---|---|---|
| Component | Dosag e | Component | Dosag e | Component | Dosag e | Component | Dosag e |
| T4 PNK | 5U | T4 PNK | 5U | T4 PNK | 5U | T4 PNK | 5U |
| BSA | 15 µg | BSA | 15 µg | BSA | 15 µg | BSA | 15 µg |
| T4 GP32 | 5 µg | E.coli SSB | 5 µg | T4 GP32 | 5 µg | T4 GP32 | 5 µg |
| Vvn | 10 ng | dsDNase | 0.5 U | SAN | 0.3 U | DNaseI | 0.3 U |
| T7 endonuclea se | 0.5 U | | | | | | |
| Taq DNA polymerase | 1.5 U | Taq DNA polymerase | 1.5 U | Taq DNA polymerase | 1.5 U | Taq DNA polymerase | 1.5 U |
| Klenow | 5U | Klenow | 25 U | *E. coli* DNA polymerase I | 3 U | T4 DNA polymerase | 45 U |
| Glycerin supplement ed to | 10 µL | Glycerin supplement ed to | 10 µL | Glycerin supplement ed to | 10 µL | Glycerin supplement ed to | 10 µL |

**Table 3. Enzyme reaction solution**

| Combination No. | Reaction buffer + Enzyme composition |
|---|---|
| Combination 1 | Reaction buffer A + Enzyme composition 1 |
| Combination 2 | Reaction buffer A + Enzyme composition 2 |
| Combination 3 | Reaction buffer A + Enzyme composition 3 |
| Combination 4 | Reaction buffer A + Enzyme composition 4 |
| Combination 5 | Reaction buffer B + Enzyme composition 1 |
| Combination 6 | Reaction buffer B + Enzyme composition 2 |
| Combination 7 | Reaction buffer B + Enzyme composition 3 |
| Combination 8 | Reaction buffer B + Enzyme composition 4 |
| Combination 9 | Reaction buffer C + Enzyme composition 1 |
| Combination 10 | Reaction buffer C + Enzyme composition 2 |
| Combination 11 | Reaction buffer C + Enzyme composition 3 |
| Combination 12 | Reaction buffer C + Enzyme composition 4 |
| Combination 13 | Reaction buffer D + Enzyme composition 1 |
| Combination 14 | Reaction buffer D + Enzyme composition 2 |
| Combination 15 | Reaction buffer D + Enzyme composition 3 |
| Combination 16 | Reaction buffer D + Enzyme composition 4 |

**Table 4. Manufacturer and Cat# of protein/enzyme**

| Name of protein/enzyme | Manufacturer | Cat# |
|---|---|---|
| T4 PNK | Vazyme | N102-01 |
| BSA | Sigma | V900933 |
| T4 GP32 | NEB | M0300L |
| Vvn | In-house expressed | See the sequence below |
| T7 Endonuclease | Vazyme | EN303 |
| Taq DNA polymerase | Vazyme | P101 |
| Klenow | Vazyme | N104 |
| dsDNase | thermo | EN0771 |
| SAN | arcticzymes | 70910 |
| DNaseI | Vazyme | EN401 |
| E.ColI SSB | abcam | ab 123224 |

Amino acid sequence of *Vibrio vulnificus* nuclease Vvn (SEQ ID NO: 1):

Ala Pro Pro Ser Thr Phe Ser Ala Ala Lys Gln Gln Ala Ala Lys Ile Tyr Gln Asp His Pro Ile Thr
Phe Tyr Cys Gly Cys Asp Ile Glu Trp Gln Gly Lys Lys Gly Ile Pro Asn Leu Glu Thr Cys Gly
Tyr Gln Val Arg Lys Ser Gln Thr Arg Ala Ser Arg Ile Glu Trp Glu His Val Val Pro Ala Trp Gln
Phe Gly His His Arg Gln Cys Trp Gln Lys Gly Gly Arg Lys Asn Cys Ser Lys Asn Asp Gln Gln

Phe Arg Leu Met Glu Ala Asp Leu His Asn Leu Ser Pro Ala Ile Gly Glu Val Asn Gly Asp Arg
Ser Asn Phe Asn Phe Ser Gln Trp Asn Gly Val Asp Gly Val Ser Tyr Gly Arg Cys Glu Met Gln
Val Asn Phe Lys Gln Arg Lys Val Met Pro Gln Thr Glu Leu Arg Gly Ser Ile Ala Arg Thr Tyr
Leu Tyr Met Ser Gln Glu Tyr Gly Phe Gln Leu Thr Lys Gln Gln Gln Leu Met Gln Ala Trp Asn
Lys Ser Tyr Pro Val Asp Glu Trp Glu Cys Ser Arg Asp Asp Arg Ile Ala Lys Ile Gln Gly Asn His
Asn Pro Phe Val Gln Gln Ser Cys Gln Thr Gln.

### Example 2. Fragmentation and end repair of a DNA template

In this example, salmon sperm gDNA was used as a template, and the initial inputs were 100 pg, 1 ng, 10 ng, 100 ng and 1 µg, respectively. gDNA was subjected to fragmentation, end repair, 3' A-tailing, and 5' phosphorylation using the different enzyme reaction solutions of Example 1, and in subsequent examples, the adaptor ligation reactions were performed using Rapid DNA Ligation Buffer and Rapid DNA Ligase in the Vayzme#ND607 VAHTS^{®} Universal DNA Library Prep Kit for Illumina^{®} V3, the adaptor ligation products and PCR amplification products were purified using Vazyme#N401 VAHTS^{®} DNA Clean Beads, and the purified ligation products were amplified and enriched using VAHTS HiFi Amplification Mix and PCR Primer Mix 3 for Illumina in the Vayzme#ND607 VAHTS^{®} Universal DNA Library Prep Kit for Illumina^{®} V3, wherein the adaptors used for ligation reactions were VAHTS^{®} DNA Adapters for Illumina^{®} (Vazyme #N801).

The fragmentation, end repair, 5' phosphorylation and 3' dA-tailing reaction system for the target DNA is shown in Table 5, wherein the formulation of an enzyme composition adopted one of the combinations 1 to 16 in Example 1, the input X µL of target DNA corresponded to 100 pg, 1 ng, 10 ng, 100 ng and 1 µg, respectively, the reactions conditions involved incubating at 37°C for 15 min, and at 65°C for 30 min, and the resulting fragmented products were subjected to sequencing library construction.

**Table 5. Fragmentation reaction system**

| Component | Dosage |
|---|---|
| Reaction buffer | 5 µL |
| Enzyme composition | 10 µL |
| Target DNA | X µL |
| Sterilized ddH₂O | Made up to 50 µL |

### Example 3. Construction of a sequencing library

In this example, the fragmented products prepared in Example 2 were subjected to adaptor ligation reactions, PCR amplification reactions and purification to construct a sequencing library. The adaptor ligation reaction system is shown in Table 6, and the formulated system was incubated at 20°C for 15 min for adaptor ligation; and 60 µL of VAHTS DNA Clean Beads were used to purify the ligation product, and the elution volume was 20/22.5 µL.

The purified product was subjected to PCR amplification, the system is shown in Table 7, and the conditions are shown in Table 8; and 45 µL of VAHTS DNA Clean Beads were used to purify the amplification product, and the elution volume was 20/22.5 µL.

**Table 6. Adaptor ligation reaction system**

| Component | Volume |
|---|---|
| Fragmented purified product | 50 µL |
| Ligation reaction buffer (Rapid DNA Ligation Buffer) | 25 µL |
| DNA ligase (Rapid DNA Ligase) | 5 µL |
| Adaptor (DNA Adaptor) | 5 µL |
| Sterilized ddH₂O | 15 µL |

**Table 7. PCR system**

| Component | Volume |
|---|---|
| Purified adaptor ligation product | 20 µL |
| Enzyme and its buffer for PCR amplification (VAHTS HiFi Amplification Mix) | 25 µL |
| Primer (PCR Primer Mix 3 for Illumina) | 5 µL |

**Table 8. PCR reaction procedure**

| Temperature | Time |
|---|---|
| 95°C | 3 min |
| 98°C | 20 sec |
| 60°C | 15 sec |
| 72°C | 30 sec |
| 72°C | 5 min |
| 4°C | Preservation |

The adaptor dilution folds and amplification cycle numbers corresponding to different initial DNA inputs are shown in Table 9.

**Table 9. Adaptor dilution folds and amplification cycle numbers corresponding to different initial DNA inputs**

| gDNA input | Adaptor dilution fold | Amplification cycle numbers |
|---|---|---|
| 100 pg | 200 folds | 14 |
| 1 ng | 100 folds | 12 |
| 10 ng | 10 folds | 8 |
| 100 ng | Stock solution | 3 |
| 1 µg | Stock solution | 2 |

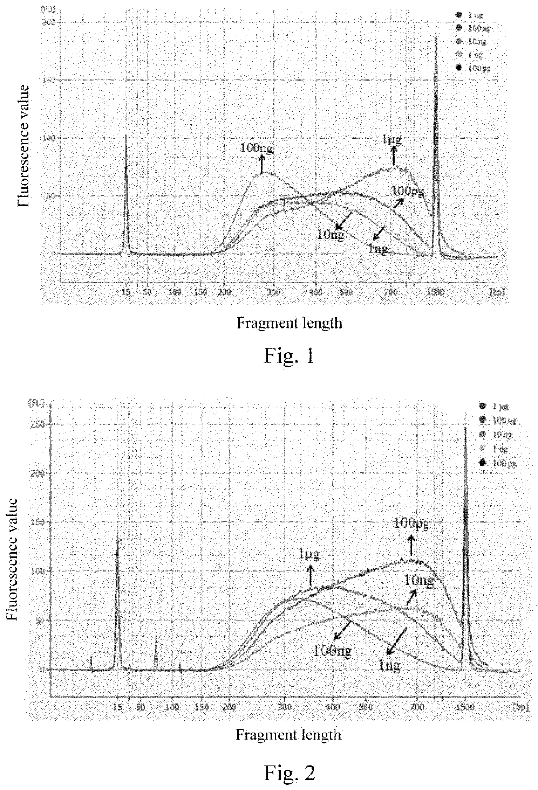

An Agilent 2100 DNA1000 chip was used to detect the length distribution of the DNA library. The results are shown in Fig. 1 to Fig. 16. It can be seen that each group of enzyme reaction solutions can achieve effective cleavage, end repair, 5' phosphorylation modification, and 3' dA-tailing to DNAs with different inputs within the same treatment time.

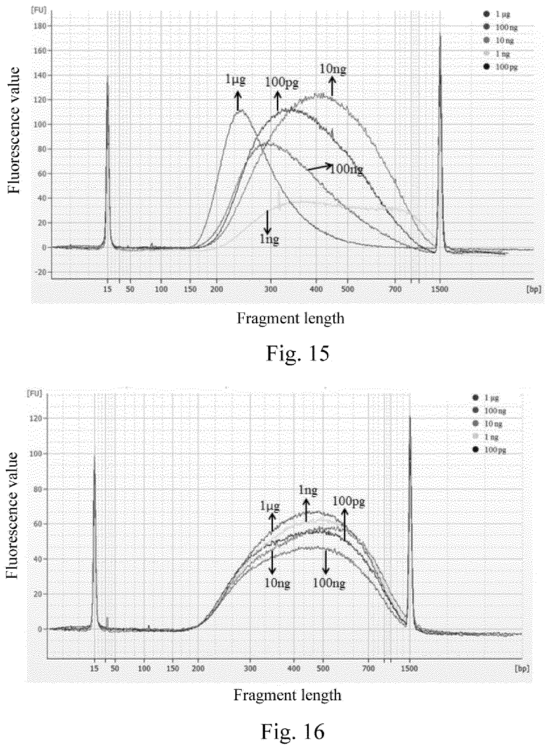

As shown in Fig. 16, the enzyme reaction solution of combination 16 has the best effect, and is fully compatible with the initial DNA input of 100 pg to 1 µg; the distribution range of library fragment lengths has good consistency, mainly in the range of 400 to 500 bp, indicating that when the enzyme composition comprises 0.3 U DNaseI, 45 U T4 DNA polymerase, 1.5 U Taq DNA polymerase, 5 U T4 PNK, 15 µg BSA, and 5 µg T4 GP32, it cooperates with the reaction buffer (300 mM Tris, 150 mM MgCl₂, 30 mM CaCl₂, 100 mM NaCl, 1 mM MnCl₂, 1 mM dNTPs, 6 mM dATP, and 50 mM ATP) to perfectly balance the enzyme digestion reaction rate and the end repair reaction rate, thereby preparing a sequencing library with a consistent fragment length.

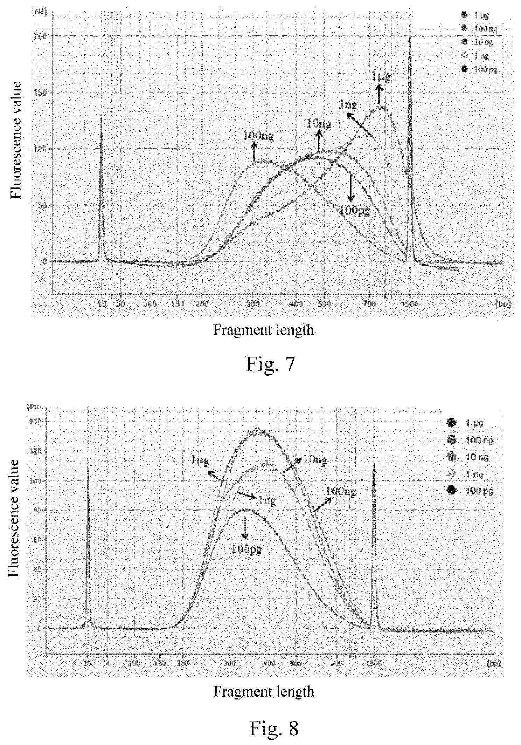

Furthermore, as shown in Fig. 8, Fig. 11 and Fig. 4, the enzyme reaction solutions of combination 8, combination 11 and combination 4 also have good effects, and have good compatibility with DNAs with an initial input of 100 pg to 1 µg, and the lengths of the prepared fragments are substantially consistent, indicating that when the enzyme composition comprises 0.004 to 0.008 U/µL endonuclease, 0.05 to 0.9 U/µL DNA polymerase, 0.01 to 0.05 U/µL Taq DNA polymerase, 0.05 to 0.3 U/µL T4 PNK, and a 0.2 to 0.6 µg/µL accessory protein, it cooperates with the reaction buffer (100 to 400 mM buffer medium, 100 to 300 mM Mg²⁺, 1 to 6 mM Mn²⁺, 50 to 250 mM Na⁺, 5 to 50 mM Ca²⁺, 0.5 to 5 mM dNTPs, 1 to 10 mM dATP, and 20 to 40 mM ATP) to regulate the enzyme digestion reaction rate and the end repair reaction rate, so that the fragment lengths of the prepared sequencing library are relatively consistent.

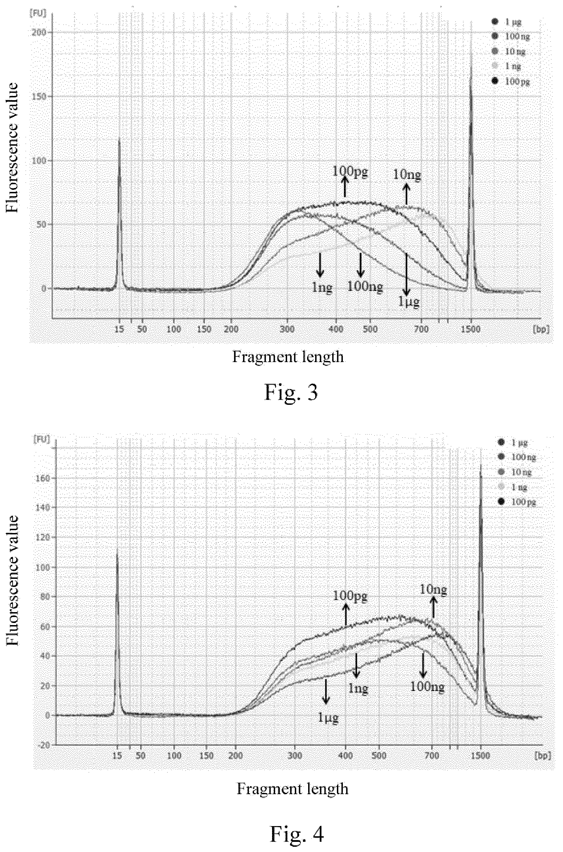

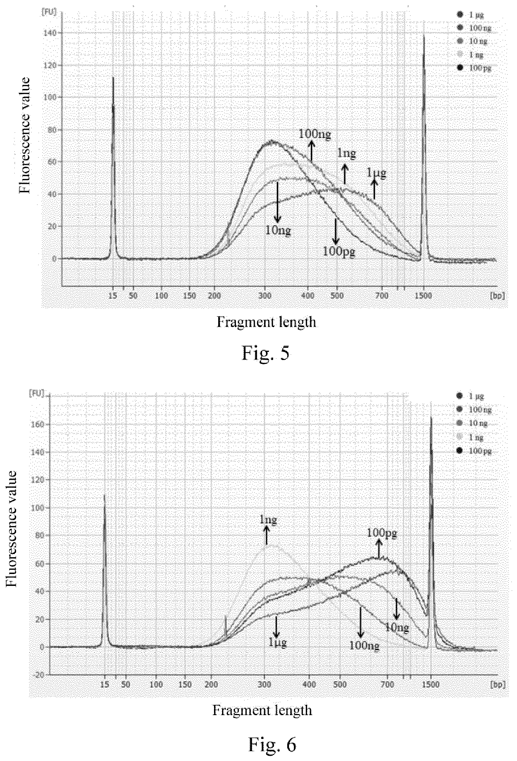

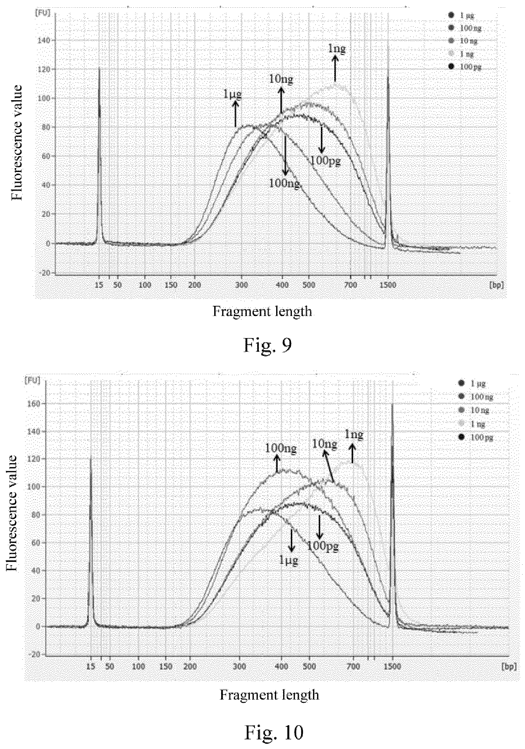

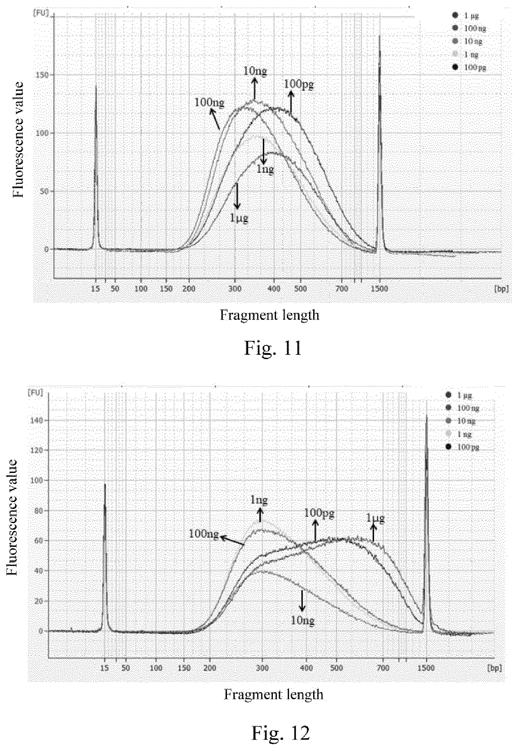

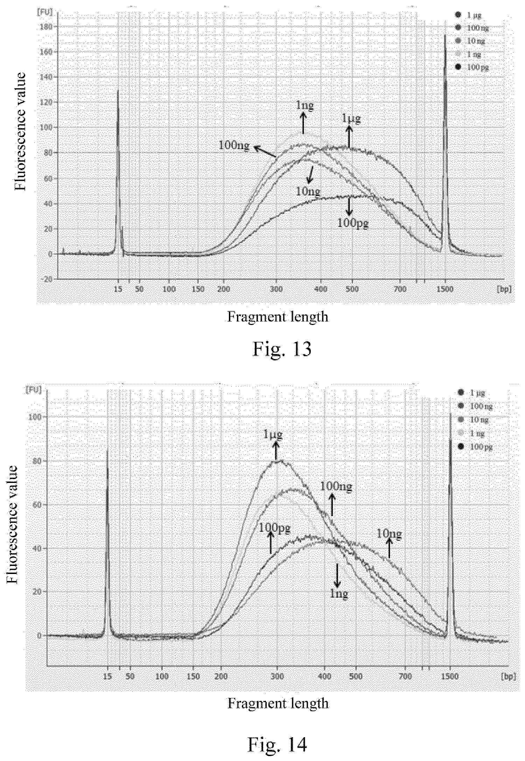

As can be seen from Fig. 1, the suitable initial input of the enzyme reaction solution of combination 1 is 100 pg to 10 ng, and further increase of the initial input will affect the fragment length; as can be seen from Fig. 2, the suitable initial input of the enzyme reaction solution of combination 2 is 100 ng to 1 µg; as can be seen from Fig. 3, the suitable initial input of the enzyme reaction solution of combination 3 is 1 ng to 10 ng; as can be seen from Fig. 5, the suitable initial input of the enzyme reaction solution of combination 5 is 100 pg to 100 ng; as can be seen from Fig. 6, the suitable initial input of the enzyme reaction solution of combination 6 is 10 ng to 100 ng; as can be seen from Fig. 7, the suitable initial input of the enzyme reaction solution of combination 7 is 100 pg to 10 ng; as can be seen from Fig. 9, the suitable initial input of the enzyme reaction solution of combination 9 is 100 pg to 10 ng; as can be seen from Fig. 10, the suitable initial input of the enzyme reaction solution of combination 10 is 100 pg to 10 ng; as can be seen from Fig. 12, the suitable initial input of the enzyme reaction solution of combination 12 is 1 ng to 100 ng; as can be seen from Fig. 13, the suitable initial input of the enzyme reaction solution of combination 13 is 1 ng to 100 ng; and as can be seen from Fig. 15, the suitable initial input of the enzyme reaction solution of combination 15 is 10 ng to 100 ng.

In summary, by optimizing the formulation of the enzyme reaction solution, through the mutual cooperation of the enzyme composition and the reaction buffer, the present application optimizes the enzyme digestion reaction rate and the end repair reaction rate in the library construction process, achieves the technical effect of obtaining a sequencing library of a consistent length under the condition of different initial amounts of samples and the same treatment time, and has wide applicability and convenient operability.

The applicant declares that the present application illustrates the detailed method of the present application through the above-mentioned examples, but the present application is not limited to the above-mentioned detailed method, that is, it does not mean that the present application must rely on the above-mentioned detailed method for implementation. Those skilled in the art should understand that any improvement to the present application, the equivalent replacement of each raw material for producing the product of the present application and the addition of auxiliary components, the selection of specific methods, and the like, all fall within the scope of protection and disclosure of the present application.

## Claims

1. An enzyme reaction solution for constructing a sequencing library, comprising an enzyme composition and a reaction buffer; wherein
the enzyme composition comprises an endonuclease, a DNA polymerase, and a polynucleotide kinase; and
the reaction buffer comprises a metal salt, a substrate, and an aqueous buffer medium solution.

2. The enzyme reaction solution according to claim 1, wherein the endonuclease comprises any one or a combination of at least two of endonuclease dsDNase, T7 endonuclease, salt-active endonuclease SAN, endonuclease Vvn or endonuclease DNaseI.

3. The enzyme reaction solution according to claim 1 or 2, wherein the DNA polymerase comprises a low-temperature DNA polymerase and/or a thermostable DNA polymerase.

4. The enzyme reaction solution according to any of claims 1 to 3, wherein the low-temperature DNA polymerase comprises any one or a combination of at least two of T4 DNA polymerase, T7 DNA polymerase, DNA polymerase I, or the large fragment Klenow of DNA polymerase I.

5. The enzyme reaction solution according to any of claims 1 to 4, wherein the thermostable DNA polymerase comprises Taq DNA polymerase.

6. The enzyme reaction solution according to any of claims 1 to 5, wherein the polynucleotide kinase comprises T4 polynucleotide kinase.

7. The enzyme reaction solution according to any of claims 1 to 6, wherein the enzyme composition further comprises an accessory protein;
optionally, the accessory protein comprises a bovine serum albumin and/or a single-chain binding protein; and
optionally, the single-chain binding protein comprises an *E. coli* single-chain binding protein and/or a T4 bacteriophage single-chain binding protein.

8. The enzyme reaction solution according to any of claims 1 to 7, wherein the metal salt comprises a metal cation, comprising any one or a combination of at least two of Mg²⁺, Mn²⁺, Na⁺ or Ca²⁺.

9. The enzyme reaction solution according to any of claims 1 to 8, wherein the substrate comprises any one or a combination of at least two of dNTPs, dATP or ATP.

10. The enzyme reaction solution according to any of claims 1 to 9, wherein the buffer medium comprises 4-hydroxyethyl piperazine ethanesulfonic acid and/or tris(hydroxymethyl)aminomethane.

11. The enzyme reaction solution according to any of claims 1 to 10, wherein the final concentration of the endonuclease in the enzyme reaction solution is 0.003 to 0.05 U/µL or 0.1 to 0.5 ng/µL;
optionally, the final concentration of the low-temperature DNA polymerase in the enzyme reaction solution is 0.01 to 0.05 U/µL;
optionally, the final concentration of the thermostable DNA polymerase in the enzyme reaction solution is 0.03 to 1.2 U/µL;
optionally, the final concentration of the polynucleotide kinase in the enzyme reaction solution is 0.05 to 0.2 U/µL;
optionally, the final concentration of the accessory protein in the enzyme reaction solution is 0.05 to 1 µg/µL;
optionally, the final concentration of the Mg²⁺ in the enzyme reaction solution is 0 to 20 mM;
optionally, the final concentration of the Mn²⁺ in the enzyme reaction solution is 0.05 to 1 mM;
optionally, the final concentration of the Na⁺ in the enzyme reaction solution is 0 to 50 mM;
optionally, the final concentration of the Ca²⁺ in the enzyme reaction solution is 0 to 10 mM;
optionally, the final concentration of the dNTPs in the enzyme reaction solution is 0.05 to 0.5 mM;
optionally, the final concentration of the dATP in the enzyme reaction solution is 0.1 to 2 mM;
optionally, the final concentration of the ATP in the enzyme reaction solution is 1 to 10 mM; and
optionally, the final concentration of the buffer medium in the enzyme reaction solution is 10 to 50 mM.

12. A method for constructing a sequencing library, comprising:
(1) adding a target nucleic acid into the enzyme reaction solution according to any of claims 1 to 11, incubating, and performing fragmentation, end repair, 5' phosphorylation and 3' A-tailing of the target nucleic acid;
(2) ligating an incubation product with a sequencing adaptor; and
(3) performing PCR on a ligation product to obtain a sequencing library.

13. The construction method according to claim 12, wherein the target nucleic acid is added in an amount of 100 pg to 1 µg.

14. The construction method according to claim 12 or 13, wherein the incubation conditions involve keeping at 35 to 40°C for 10 to 20 min, and at 60 to 70°C for 20 to 40 min.

15. A kit for constructing a sequencing library, comprising the enzyme reaction solution according to any of claims 1 to 11; and
optionally, the kit further comprises any one or a combination of at least two of a sequencing adaptor, a ligation reaction reagent or a PCR reagent.
